Europäisches Patentamt

European Patent Office

Office européen des brevets

(11) Publication number: **0 484 863 A1**

## EUROPEAN PATENT APPLICATION

(21) Application number: **91118790.4**

(22) Date of filing: **04.11.91**

(51) Int. Cl.5: **C12P 21/08**, G01N 33/92

(30) Priority: **07.11.90 JP 301510/90**

(43) Date of publication of application:
**13.05.92 Bulletin 92/20**

(84) Designated Contracting States:
**AT BE CH DE DK ES FR GB GR IT LI LU NL SE**

(71) Applicant: **DAIICHI PURE CHEMICALS CO. LTD.**
**13-5, Nihonbashi 3-chome**
**Chuo-ku Tokyo(JP)**

(72) Inventor: **Kondo, Akira**
**5-22-10-201, Higashikoiwa, Edogawa-ku Tokyo(JP)**
Inventor: **Uchida, Mariko**
**1-6-5-201, Omorihigashi, Ota-ku Tokyo(JP)**
Inventor: **Manabe, Mitsuhisa**
**2-5-26-415, Shibamata, Katsushika-ku Tokyo(JP)**
Inventor: **Sakai, Yasuo**
**3-5-1-505, Hirayama**
**Inzai-machi, Inba-gun, Chiba(JP)**

(74) Representative: **Wächtershäuser, Günter, Dr.**
**Tal 29**
**W-8000 München 2(DE)**

(54) **A monoclonal antibody and a method for measuring malondialdehyde-reacted low-density-lipoproteins.**

(57) Monoclonal antibodies recognizing human malondialdehyde-modified low-density-lipoproteins (human MDA-modified-LDL) and monoclonal antibodies recognizing human MDA-modified-LDL and reduced type human MDA-modified-LDL are disclosed. They are produced by fusion cells prepared by using human MDA-modified-LDL or reQduced type human MDA-modified-LDL as an antigen. The monoclonal antibodies can be used for measurement of human MDA-modified-LDL by immobilizing either one of such monoclonal antibodies or an antibody recognizing human apo B on an insoluble carrier, labeling the other of the antibodies with an enzyme, allowing such an immobilized antibody and labeled antibody to come contact with a sample to be detected, and subjecting the sample to the sandwich enzymatic immunoassay.

EP 0 484 863 A1

BACKGROUND OF THE INVENTION

Field of the Invention:

The present invention relates to a monoclonal antibody recognizing human malondialdehyde-modified low-density-lipoproteins which are considered to be a cause of arteriosclerosis (hereinafter abbreviated as "human MDA-modified-LDL") and reduced type human MDA-modified-LDL. The present invention also relates to a method for measuring human MDA-modified-LDL using such a monoclonal antibody.

Description of the Background Art:

Arteriosclerosis is known to be a major cause of ischemic heart disease, cerebral infarct, and the like. The mechanism by which arteriosclerosis is caused is considered to be the arterial malfunction due to accumulation of smooth muscle cells, connective tissues, and a large amount of lipids, of which a majority is cholesterol esters, in the development of endotherial layers. These accumulated materials form atheromas, cause arterial walls to become thick and sclerotic, and finally obstruct normal arterial functions. The atheromas, observed in the initial stage of arteriosclerosis, are formed by foam cells which are generated due to the engulfment by macrophages ducts of denatured low-density-lipoproteins (LDL). Particulars of denatured LDL which functions to convert macropharges into foam cells in living bodies are still to be elucidated. In recent years, possibilities for two types of denatured LDL which might be involved in the initiation of arteriosclerosis has been reported; one is denatured LDL produced by the interaction between LDL and endotherial cells which are now considered to play an important roll in the development of arteriosclerosis [M. T. Quinn, S. Parthasarathy, L. G. Fong, and D. Steinberg, Proc. Natl. Acad. Sci. U.S.A., 84, 2995-2998 (1987)]. It is known that the physiological properties of the modified-LDL by endotherial cells are similar to those of the chemically oxidized LDL by copper ions. The other is LDL modified with malondialdehyde (MDA), known as the final degradation product of lipid peroxides and thromboxane $A_2$ which are also considered to play an important roll in pathogenesis of arteriosclerosis [A. M. Fogelman, I. Schechter, J. Seager, M. Hokom, J. S. Child, and P. A. Bdwards, Proc. Natl. Acad. Sci. U.S.A., 77, 2214-2218 (1980)].

On the basis of these findings, histochemical studies, using antibodies against MDA-modified-LDL and copper ions oxidized LDL, demonstrated these denatured LDLs in arteriosclerotic lesions.

These reports arose a great concern over the serum levels of denatured LDL.

At present, there are two reports on the methods which are understood to possibly measure denatured LDL in the serum of the patients with atheroma. Takano et al, in the article of Japanese Patent Kokai No. 63625/1988 provide the assay method based on monoclonal antibodies obtained by using serum from patient suffering from arteriosclerosis or homogenate of arteriosclerosis lesions as an antigen. Kalenov, in the article of Japanese Patent Kokai No. 73151/1988 provides a distinct assay method based on monoclonal antibodies obtained by using deposited fats in arterial wall of a patient suffering from atherosclerosis as an antigen

However, since antigens used in these studies consist of various kinds of antigens (not homogeneous), the substance which reacts with obtained antibodies has not been identified. In other words, the analytes which are measured by these assay methods are still unknown. This fact makes it very difficult to apply these methods to clinical use.

Moreover, these methods only enable relative evaluations by the comparison of the values of patient serum with that of serum from a healthy person, since no standard materials for the methods like these are available. These factors render the conventional assay system incomplete and unreliable.

In view of this situation, the present inventors have undertaken extensive studies and were successful in obtaining a monoclonal antibody which can detect human MDA-modified-LDL and a monoclonal antibody which can detect human MDA-modified-LDL and reduced type human MDA-modified-LDL, by the use of uniform human MDA-modified-LDL or reduced type human MDA-modified-LDL as antigens.

SUMMARY OF THE INVENTION

Accordingly, an object of the present invention is to provide a monoclonal antibody which can recognize human MDA-modified-LDL.

Another object of the present invention is to provide a monoclonal antibody which can recognize human MDA-modified-LDL and reduced type human MDA-modified-LDL.

Still another object of the present invention is to provide a method for measuring MDA-modified-LDL which comprises,

immobilizing either one of said monoclonal antibody or an antibody recognizing human apo B on an insoluble carrier,

labeling the other antibody with an enzyme,

allowing said immobilized antibody and labeled antibody to come contact with a sample to be detected, and

subjecting the sample to the sandwich enzymatic immunoassay.

Other objects, features and advantages of the invention will hereinafter become more readily apparent from the following description.

## BRIEF DESCRIPTION OF THE DRAWINGS

Figure 1 is a graph showing the reactivities of monoclonal antibodies obtained in Example 2 with human LDL.

Figure 2 is a graph showing the reactivities of monoclonal antibodies obtained in Example 2 with human MDA-modified-LDL.

Figure 3 is a graph showing the reactivities of monoclonal antibodies obtained in Example 2 with reduced human MDA-modified-LDL.

Figure 4 is a sketch showing stained bands of samples such as serum from a healthy person, purified human LDL, and prepared human MDA-modified-LDL, detected by the Western blotting method after electrophorested in SDS-PAGE.

Figure 5 is a graph showing the relationship between antigen concentrations and enzymatic activities, when human LDL, human MDA-modified-LDL, and reduced type human MDA-modified-LDL are measured by one-step sandwich enzymatic immunoassay in which a monoclonal antibody of the present invention was used.

Figure 6 is a graph showing the relationship between antigen concentrations and enzymatic activities, when human MDA-modified-LDL was measured by the two-step sandwich enzymatic immunoassay in which a monoclonal antibody of the present invention was used.

## DETAILED DESCRIPTION OF THE INVENTION AND PREFERRED EMBODIMENTS

Monoclonal antibodies which can recognize human MDA-modified-LDL, or both human MDA-modified-LDL and reduced type human MDA-modified-LDL, can be prepared, for example, according to the following procedures.

Purified LDL is first prepared from fresh human serum by a common ultra-centrifugation. Malondialdehyde (MDA) is modified wish the purified LDL to produce human MDA-modified-LDL. The reaction is preferably carried out at a pH of 6-7, a temperature of 20-40°C for 30 minutes to 24 hours. The reaction can be terminated by cooling the reaction mixture to a temperature of below 4°C. After termination of the reaction, the mixture is dialyzed at 4°C against a buffer solution with a pH 6-8 to which 10-100 $\mu$M EDTA is optionally added.

Human MDA-modified-LDL can be converted into reduced type human MDA-modified-LDL by an appropriate reducing treatment in order to improve its storability. Any reducing agents which can reduce a Schiff base can be used, with a preferred reducing agents being $NaBH_4$, $NaCNBH_3$, etc.

Anti-human MDA-modified-LDL monoclonal antibody can be produced by a known cell fusion method by using the human MDA-modified-LDL as an immune source. Specifically, a mouse is immuned with the human MDA-modified-LDL and its spleen is enucleated after a certain period of time. The spleen cells are then fused with myeloma cells and the fusion cells are cultivated for a prescribed period of time. Fusion cell lines which can produce anti-human MDA-modified-LDL monoclonal antibodies with a high specificity can be isolated, judging from antibodies produced in the supernatant by their differences in reactivity with LDL and MDA-modified-LDL.

Furthermore, fusion cell lines which can produce monoclonal antibodies recognizing reduced type human MDA-modified-LDL, in addition to human MDA-modified-LDL, can be collected with the measurement of the reactivity with reduced type human MDA-modified-LDL.

Denatured LDL has not been confirmed to exist in human serum, although the existence has been speculated by the use of an antibody recognizing arteriosclerosis lesions which are considered to contain denatured LDL.

The existence of MDA-modified-LDL in human serum was immunologically confirmed for the first time by using the above antibodies in the investigation by the present inventors. Since there is possibility that MDA-modified proteins other than MDA-modified-LDL are contained in human serum [J. F. Kergonou, E. Bruna, I. Pennacino, and R. Ducousso, Advances in the Bioscience, 71, 121-124 (1988)], the possibility of said anti-human MDA-modified-LDL monoclonal antibody to reaction with such other MDA-modified proteins must be taken into account. Therefore, in order to promote the specificity in the measurement of human MDA-modified-LDL, the sandwich enzymatic immunoassay by using an antibody which can recognize human apo B constituting human MDA-modified-LDL and LDL is desirable.

Specifically, the method for measuring of MDA-modified-LDL according to the present invention comprises: immobilizing either (i) one of monoclonal antibodies which can recognize human MDA-modified-LDL or monoclonal antibodies which can recognize human MDA-modified-LDL and reduced type human MDA-modified-LDL, or (ii) an antibody recognizing human apo B, on an insoluble carrier; labeling the other with an enzyme; allowing said immobilized antibody and labeled antibody to come contact with a sample to be detected; and detecting the MDA-modified-LDL by the sandwich enzymatic immunoassay.

Insoluble carriers for immobilizing monoclonal antibodies used in the present invention may be a synthetic polymer of various types such as polyethylene, polypropylene, etc., glass, silicon, an insoluble polysaccharide, or the like. A carrier can be used in the form of spheres, rods, fine particles, or as a test tube or a microtitre plate. Monoclonal antibodies can be immobilized by physically adsorbing them into a carrier or binding them to a carrier by a covalent bond.

An enzyme-labelled antibody can be prepared according to a known method. Optionally, the antibody to be used may be partly degraded by a suitable protease, or converted into F(ab')$_2$ or Fab' in the presence of a reducing agent before labeled with an enzyme. $\beta$-D-Galacsidase, peroxidase, alkaline phosphatase, glucose oxidase, and the like are given as examples of enzymes by which the monoclonal antibody is labeled.

In carrying out the immunoreaction, in the first step, a sample is contacted with the insoluble antibody to bind the antigen to the carrier, thus producing a complex of insoluble antibodies and antigens; and, in the second step, the complex of insoluble antibodies and antigens is combined with the enzyme-labeled antibody to produce a complex of insoluble antibodies, antigens, and enzyme-labeled antibodies; provided that a one-step immunoreaction is possible for the selection of a fusion cell producing an anti-human MDA-modified-LDL monoclonal antibody, since a purified antigen is used in the selection of such a monoclonal antibody. The amount of antigen in a sample can be determined by the measurement of the enzymatic activity of the complex thus obtained.

Other features of the invention will become apparent in the course of the following description of the exemplary embodiments which are given for illustration of the invention and are not intended to be limiting thereof.

EXAMPLES

Example 1 〈Preparation of Human MDA-modified-LDL and Reduced Type Human MDA-modified-LDL〉

Purified human LDL (3 mg protein/ml) and MDA (sodium salt; 66.7 mM) were reacted in 50 mM phosphate buffer (pH 6.5) at 37°C for 6 hours. After the reaction, the resulting product was dialyzed against a $Ca^{2+}$, $Mg^{2+}$-free Dulbecco's phosphate-buffered saline (pH 7.4) containing 100 $\mu$M EDTA at 4°C for 24 hours to obtain human MDA-modified-LDL.

For the production of reduced type human MDA-modified-LDL, after determination of the protein content of the human MDA-modified-LDL, the human MDA-modified-LDL (0.5 mg protein/ml) and 25 mM NaBH$_4$ were reacted in the above Dulbecco's phosphate-buffered saline (pH 7.4) at 37°C for 3 hours. After the reaction, the resulting product was dialyzed against the Dulbecco's phosphate-buffered saline (pH 7.4) at 4°C for 24 hours to obtain reduced type human MDA-modified-LDL.

Example 2 〈Preparation of Monoclonal Antibody Recognizing Human MDA-modified-LDL〉

10 $\mu$g of the human MDA-modified-LDL was thoroughly blended with a complete Freund's adjuvant and intraperitoneally injected into a BALB/c mouse. The injection was repeated twice, one time in two weeks, to immunize the mouse. After one week following the final injection, 10 $\mu$g of the human MDA-modified-LDL dissolved in 100 $\mu$l of physiological saline was injected through the caudal vein. Three days thereafter, the spleen was extracted, well loosened, and washed with a medium (PRMI 1640). The spleen cells ($2.5 \times 10^8$) thus prepared were mixed with myeloma cells ($2.5 \times 10^7$) of a SP2/0•Ag14 mouse, which were thoroughly

4

washed with the medium in the same manner. The mixture (0.25 ml) was slowly dropped into a medium (50 w/v% PEG1540). After blending the mixture for 1 minute, PEG was diluted by slowly adding 8 ml of GKN medium to terminate the reaction. The resulting mixture was centrifuged for 5 minutes at 1,500 rpm to collect cells. The cells were washed with 2 ml of the medium and suspended into 30 ml of HAT medium. The suspension was inoculated into a 96-hole microplate, 0.1 ml for each well, and incubated at 37°C in 8% $CO_2$ for 10 days. 0.2 ml of the supernatant of each well was removed and replaced with 0.2 ml of HT medium (the same medium as HAT medium, but not containing aminopterin). The antibody titer was determined on the supernatant which was obtained after the procedure of the supernatant removal and the addition of HT medium was repeated three times. Cells with a strong antibody activity were cloned by means of the limiting dilution method, finally obtaining 18 isolated fusion cell lines. The fusion cells were intraperitoneally injected to a BALB/c mouse which was treated with pristane. After 10-20 days, ascites fluid of the mouse was collected to obtain monoclonal antibodies therefrom. The classes and subclasses were determined on the monoclonal antibodies. The results are shown in Table 1.

TABLE 1

| Antibody No. | Class | Subclass | L-chain | Group |
|---|---|---|---|---|
| 29208 | G | 2b | *Kappa* | A |
| 29209 | G | 1 | *Kappa* | A |
| 29210 | G | 1 | *Kappa* | A |
| 29211 | G | 1 | *Kappa* | A |
| 29212 | G | 1 | *Kappa* | A |
| 29213 | G | 1 | *Kappa* | B |
| 29214 | G | 1 | *Kappa* | A |
| 29215 | G | 1 | *Kappa* | B |
| 29217 | G | 2a | *Kappa* | B |
| 29218 | G | 1 | *Kappa* | B |
| 29219 | G | 1 | *Kappa* | A |
| 29220 | G | 1 | *Kappa* | A |
| 29221 | G | 1 | *Kappa* | A |
| 29222 | G | 1 | *Kappa* | B |
| 29223 | G | 2a | *Kappa* | A |
| 29224 | G | 1 | *Kappa* | B |
| 29225 | G | 1 | *Kappa* | A |
| 29226 | G | 2a | *Kappa* | B |

In order to determine the difference of the antigenic determinant by those antibodies, each antibody was labeled with peroxidase and subjected to the reactive competition with that unlabeled antibody to human MDA-modified-LDL. Resulting from the presence or absence of the reactive composition between both antibodies, all kinds of antibodies were classified by those recognition sites into two groups A and B as indicated in Table 1.

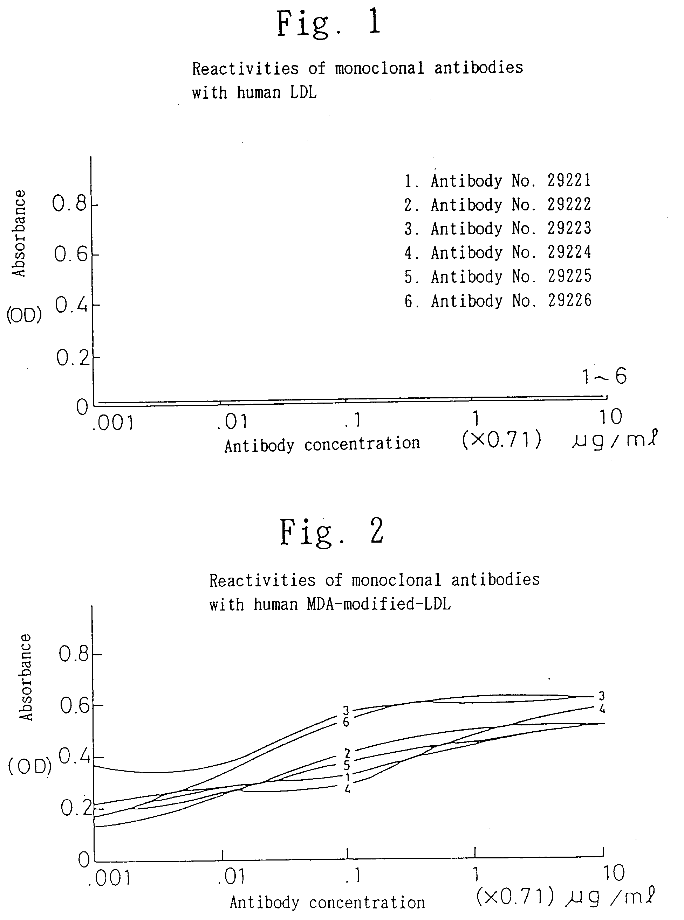

The reactivity of some of the antibodies to human LDL, human MDA-modified-LDL, and reduced type human MDA-modified-LDL were determined according to the following procedure. The antigens (human LDL, human MDA-modified-LDL, or reduced type human MDA-modified-LDL were dissolved in a phosphate buffered saline (pH 7.2) (hereinafter abbreviated as PBS) at a concentration of 2 $\mu$g/ml respectively and aliquots of 50 $\mu$l/well were added into a microplate, to immobilize at 4°C overnight. The wells were thoroughly washed with PBS to which 1% bovine serum albumin (BSA) and 0.05% Tween 20 were added (such a PBS is herein abbreviated as PBS-BSA). To the wells were added antibodies dissolved in BSA-PBS at a concentration of 7.1 $\mu$g/ml and its diluents, 4 solutions diluted with BSA-PBS by a factor of 10, in an amount of 50 $\mu$l per each well, and reacted at 37°C for 1 hour. After washing, was added peroxidase-labeled anti-mouse IgG(Fc) goat antibody diluted to 1,000-fold with BSA-PBS, in an amount of 50 $\mu$l per each well, and reacted at 37°C for 1 hour. After washing, an enzymatic reaction was carried out using hydrogen peroxide and orthophenylenediamine as substrates. The activity is detected by the absorbance at 550 nm. The results are shown in Figures 1-3.

Example 3 〈Confirmation of Substances Reacting to Monoclonal Antibody Against Human MDA-modified-LDL in Normal Human Serum〉

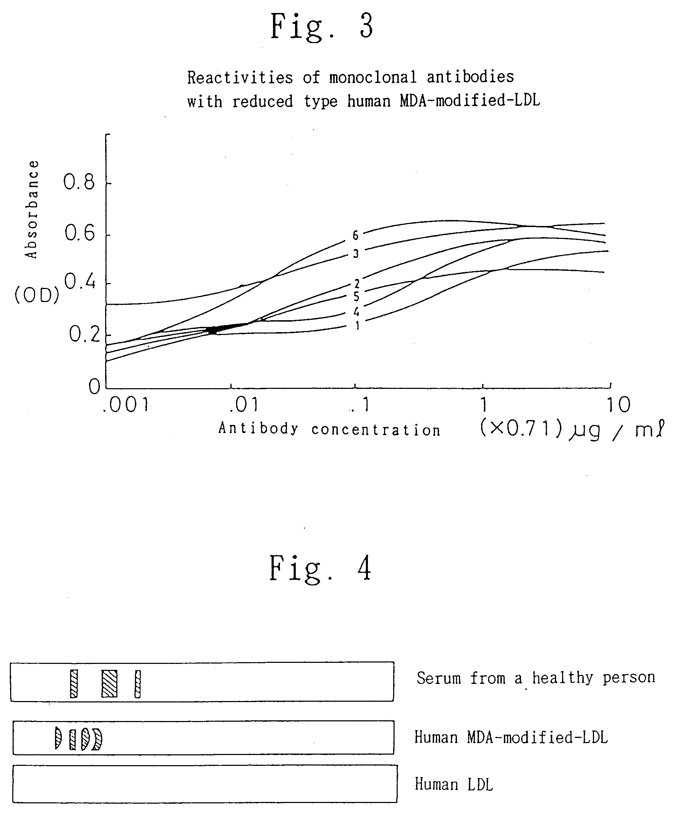

Normal human serum electrophorated with polyacrylamide gel to which SDS was added and electrically blotted on a polyvinylidene difluoride membrane. The membrane was allowed to stand still in a phosphate buffer solution (pH 7.2) containing 10% skim milk at 4°C overnight. After washing the membrane with a phosphate buffer solution (pH 7.2) containing 0.02% Tween 20 and 1% BSA, the serum was reacted at room temperature for 1 hour with an antibody No. 29209, as a primary antibody, and at room temperature for 1 hour with a 500-fold dilute of peroxidase-labeled anti-mouse IgG rabbit antibody, as a secondary antibody. After thoroughly washing the membrane, an enzymatic reaction was carried out using hydrogen peroxide and 3,3'-diaminobenzidine hydrochloride as substrates. The results are shown in Figure 4, which indicates several stained bands which are considered to be MDA-modified apo B protein. Beside these bands, were confirmed several bands which are considered to be other MDA-modified proteins.

Example 4 〈Measurement of Human MDA-modified-LDL by the One-step Sandwich Enzymatic Immunoassay〉

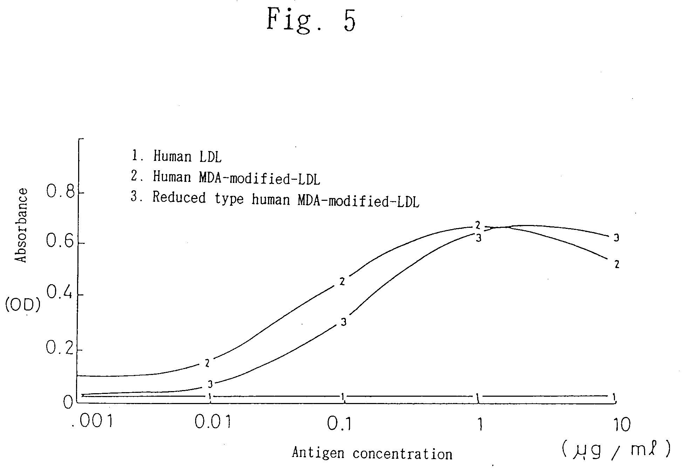

Antibody No. 29210 was diluted with PBS to a concentration of 10 mOD, added into a microplate in an amount of 50 $\mu$l/well, and immobilized by allowing to stand still at 4°C overnight. To the wells were added antibodies, human LDL, human MDA-modified-LDL, or reduced human MDA-modified-LDL, each dissolved in BSA-PBS at a concentration of 10 $\mu$g/ml and their diluents, 4 solutions diluted with BSA-PBS by a factor of 10, in an amount of 50 $\mu$l per each well, and reacted at 37°C for 1 hour. After washing, was added peroxidase-labeled anti-mouse IgG(Fc) goat antibody diluted to 1,000-fold with BSA-PBS, in an amount of 50 $\mu$l per each well, and reacted at 37°C for 1 hour. After washing, an enzymatic reaction was carried out using hydrogen peroxide and orthophenylenediamine as substrates. The enzymatic activity was measured and detected by the absorbance at 550 nm. The results are shown in Figure 5.

Example 5 〈Measurement of Human MDA-modified-LDL by the Two-step Sandwich Enzymatic Immunoassay〉

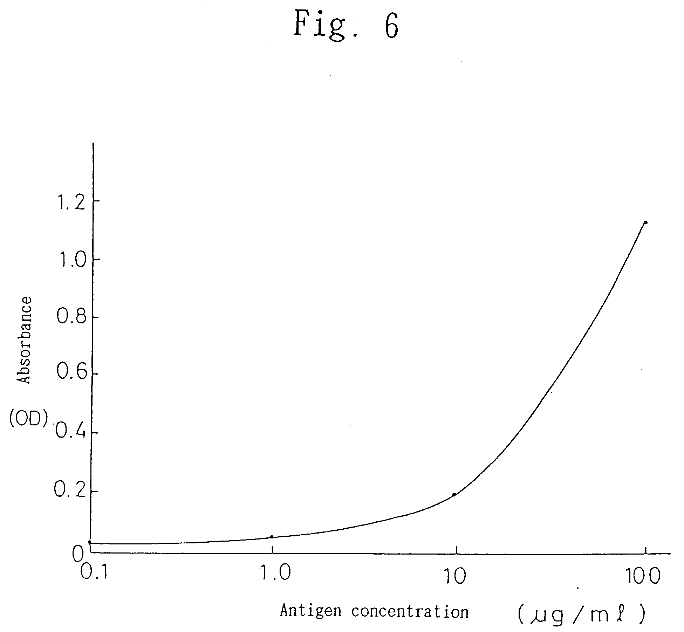

Antibody No. 29209 was immobilized on polystyrene ball (a diameter of 1/4"), into antibody 4 mOD per ball in a carbonate buffer solution (pH 9.6), by allowing to stand still at 4°C for 48 hours, followed by the reaction with antibody-bound balls from after subjected to blocking with BSA-PBS at 25°C for 48 hours. For the measurement, human MDA-modified-LDL dissolved in BSA-PBS at a concentration of 10 $\mu$g/ml and their diluents, 3 solutions diluted with BSA-PBS by a factor of 10, were added into test tubes (20 $\mu$l/tube). 500 $\mu$l of BSA-PBS and then above antibody-bound balls were added to each tube, and reacted at 37°C for 1 hour. After thoroughly washing, peroxidase-labeled anti-apo B monoclonal antibody fluid was added to test tubes (500 $\mu$l/tube), and reacted at 37°C for 1 hour. After washing, an enzymatic reaction was carried out using hydrogen peroxide and orthophenylenediamine as substrates. The enzymatic activity was measured and detected by the absorbance at 492 nm. The results are shown in Figure 6.

Obviously, numerous modifications and variations of the present invention are possible in light of the above teachings. It is therefore to be understood that within the scope of the appended claims, the invention may be practiced otherwise than as specifically described herein.

**Claims**

1. A monoclonal antibody recognizing human malondialdehyde-modified low-density-lipoproteins.

2. A monoclonal antibody recognizing human malondialdehyde-modified low-density-lipoproteins and reduced type human malondialdehyde-modified low-density-lipoproteins.

3. A method for measuring human malondialdehyde-modified low-density-lipoproteins which comprises,
   immobilizing either (i) one of monoclonal antibodies defined in Claim 1 or 2, or (ii) an antibody recognizing human apo B on an insoluble carrier,
   labeling the other of the antibodies (i) or (ii) with an enzyme,
   allowing said immobilized antibody and labeled antibody to come contact with a sample to be detected, and
   subjecting the sample to the sandwich enzymatic immunoassay.

# Fig. 1

Reactivities of monoclonal antibodies
with human LDL

1. Antibody No. 29221
2. Antibody No. 29222
3. Antibody No. 29223
4. Antibody No. 29224
5. Antibody No. 29225
6. Antibody No. 29226

# Fig. 2

Reactivities of monoclonal antibodies
with human MDA-modified-LDL

# Fig. 3

Reactivities of monoclonal antibodies
with reduced type human MDA-modified-LDL

# Fig. 4

Serum from a healthy person

Human MDA-modified-LDL

Human LDL

# Fig. 5

Fig. 6

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | ATHEROSCLEROSIS vol. 65, no. 3, June 1987, SHANNON, IRELAND pages 265 - 272; B. GONEN ET AL.: 'Immunogenicity of malondialdehyde-modified low density lipoproteins' * abstract * | 1,2 | C12P21/08 G01N33/92 |
| Y | | 3 | |
| Y | EP-A-0 339 632 (KYOWA HAKKO KOGYO KABUSHIKI KAISHA) * the whole document * | 3 | |
| X | ARTERIOSCLEROSIS vol. 10, no. 3, May 1990, DALLAS, USA pages 325 - 335; W. PALINSKI ET AL.: 'Antisera and monoclonal antibodies specific for epitopes generated during oxidative modification of low density lipoprotein.' * page 329, left column, line 15 - right column, line 8 * | 1,2 | |
| A | AMERICAN JOURNAL OF PATHOLOGY vol. 135, no. 5, November 1989, HAGGERSTOWN MD, USA pages 815 - 825; H. BOYD ET AL.: 'Direct evidence for a protein recognized by a monoclonal antibody against ixidatively modified LDL in atherosclerotic lesions from a Watanabe heritable hyperlipidemic rabbit.' * the whole document * | 1,2 | TECHNICAL FIELDS SEARCHED (Int. Cl.5 ) C07K C12P G01N |
| X,P | EP-A-0 433 088 (ELI LILLY AND COMPANY) * the whole document * | 1-3 | |

The present search report has been drawn up for all claims

| Place of search | Date of completion of the search | Examiner |
|---|---|---|
| THE HAGUE | 11 FEBRUARY 1992 | NOOIJ F.J.M. |